# EUROPEAN PATENT SPECIFICATION

(11) **EP 2 141 180 B1**
(45) Date of publication and mention of the grant of the patent: **31.07.2013**
(21) Application number: 09164101.9
(22) Date of filing: 30.06.2009
(51) Int. Cl.: G01N 33/68, C07K 16/18

(54) **Antibody recognizing canine CRP and human CRP**
Antikörpererkennung gegen Hunde-CRP und humanes CRP
Anticorps reconnaissant le CRP canin et CRP humain

(30) Priority: 30.06.2008 JP 2008170284
(43) Date of publication of application: 06.01.2010
(73) Proprietor: FUJIFILM Corporation, Tokyo 106-0031 (JP)
(72) Inventor: Masuda, Nobuhito, Saitama 351-8585 (JP); Ikeda, Morihito, Kanagawa 258-8577 (JP); Kawasaki, Kazuya, Saitama 351-8585 (JP)
(74) Representative: HOFFMANN EITLE

(56) References cited:
- YAMAMOTO S ET AL: "Canine C-reactive protein (CRP) does not share common antigenicity with human CRP" VETERINARY RESEARCH COMMUNICATIONS, vol. 17, no. 4, 1993, pages 259-266, XP009123084 ISSN: 0165-7380
- KJELGAARD-HANSEN MAD ET AL: "Internal quality control of a turbidimetric immunoassay for canine serum C-reactive protein based on pooled patient samples." VETERINARY CLINICAL PATHOLOGY / AMERICAN SOCIETY FOR VETERINARY CLINICAL PATHOLOGY 2004, vol. 33, no. 3, 2004, pages 139-144, XP002546867 ISSN: 0275-6382
- KJELGAARD-HANSEN MADS ET AL: "Evaluation of a commercially available human C-reactive protein (CRP) turbidometric immunoassay for determination of canine serum CRP concentration." VETERINARY CLINICAL PATHOLOGY / AMERICAN SOCIETY FOR VETERINARY CLINICAL PATHOLOGY 2003, vol. 32, no. 2, 2003, pages 81-87, XP002546868 ISSN: 0275-6382
- YAMAMOTO S ET AL: "Efficient preparation of monospecific anti-canine C-reactive protein serum and purification of canine C-reactive protein by affinity chromatography" VETERINARY IMMUNOLOGY AND IMMUNOPATHOLOGY, AMSTERDAM, NL, vol. 36, no. 3, 1 April 1993 (1993-04-01), pages 293-301, XP023689481 ISSN: 0165-2427 [retrieved on 1993-04-01]
- FRANSSON BOEL A ET AL: "Assessment of three automated assays for C-reactive protein determination in dogs" AMERICAN JOURNAL OF VETERINARY RESEARCH, vol. 68, no. 12, December 2007 (2007-12), pages 1281-1286, XP009123080 ISSN: 0002-9645
- KJELGAARD-HANSEN M ET AL: "Canine serum C-reactive protein detected by means of a near-patient test for human C-reactive protein." THE JOURNAL OF SMALL ANIMAL PRACTICE JUN 2008, vol. 49, no. 6, June 2008 (2008-06), pages 282-286, XP002546870 ISSN: 0022-4510

## Description

### Technical Field

The present invention relates to a monoclonal antibody recognizing both canine CRP and human CRP, a hybridoma which produces the aforementioned monoclonal antibody, and a reagent and a method for measuring canine CRP and human CRP using the aforementioned monoclonal antibody.

### Background Art

C-reactive protein (CRP) has been known as a protein whose production amount correlates with the strength of an inflammatory response. Thus, a value obtained by measuring the amount of CRP in serum can be used as an indicator of such inflammatory response. That is to say, if the serum CRP value is high, it shows strong inflammation.

JP Patent Publication (Kokai) No. 62-210984 A (1987) discloses that a known technique of producing a monoclonal antibody is applied to production of an anti-canine CRP monoclonal antibody, and that the obtained antibody is applied to purification of an antigen (canine CRP) according to a known affinity chromatography technique. The obtained antibody is characterized in that it is able to capture canine CRP, but it is not used in quantification of canine CRP. In addition, the above publication does not describe that this antibody can recognize human CRP.

Vet. Clin. Pathol. 2003, 32: 81-87 describes that an antibody used in a test kit for immunonephelometry, which is used to measure human CRP, has been accidentally found to be reactive with canine CRP. This publication neither suggests that the used antibody has been prepared while its reactivity with canine CRP has been taken into account, nor describes that this antibody is a monoclonal antibody. A method for stably producing this antibody is not described in this publication, either.

Japanese Patent No.3151080 describes a dry immunoanalytical element used in the measurement of human CRP. However, this publication does not describe its reactivity with canine CRP. As a matter of fact, even if canine serum is measured, the results do not show quantitative property. Thus, it cannot be used in quantification of canine CRP. This is because the specificity of the antibody used in Japanese Patent No.3151080 is limited to human CRP and because the aforementioned antibody does not substantially recognize canine CRP.

YAMAMOTO S ET AL: "Canine C-reactive protein (CRP) does not share common antigenicity with human CRP", VETERINARY RESEARCH COMMUNICATIONS, vol. 17, no. 4, 1993, pages 259-266, discloses that canine and human CRP do not share common antigenicity.

### Disclosure of the Invention

In known methods for producing novel monoclonal antibodies, the largest amount of labor is required for an operation to select (screen) fused cells (antibody-producing hybridomas) of antibody-producing cells obtained from an immunized animal after immunization and cells having infinite proliferative capacity (myeloma cells). In an ELISA method using a microplate that is a common screening method, only limited information of the characteristics of an antibody, such as "whether or not the antibody reacts with an antigen used in screening" is reflected. Even if the antibody is determined to "react with such antigen," it is impossible to determine the dominance of the two parties. In many cases, a coupling constant, which is a particularly important characteristic of an antibody, is generally measured, after the selected hybridomas have been further cultured and acclimatized to establish clones, and a purified antibody is then obtained by culture or sensitization to animals. Thus, at the time of selection of hybridomas, high bindability is not guaranteed.

In the present invention, an attempt has been made to precisely select and obtain a hybridoma that produces a high-affinity antibody recognizing both canine CRP and human CRP by selecting such hybridoma while focusing on the subclass, antigenic specificity, and coupling constant of an antibody produced by the hybridoma at the time of selection of the hybridoma. If the hybridoma-derived antibody obtained by this method is applied to various types of immunoanalytical principles, there can be provided a measurement reagent and a measurement method, which are capable of measuring both canine CRP and human CRP using a single analytical reagent. For example, a reagent is prepared by sensitizing latex with this antibody. After the reagent has been allowed to come into contact with serum containing canine CRP or human CRP, etc., the aggregation state of the latex is measured by an optical method such as turbidimetry, so that the concentration of canine CRP or human CRP contained therein can be quantified.

Moreover, a complex of the antibody fragment described in Japanese Patent No. 3151080 and *Bacillus subtilis* α-amylase is produced using an antibody derived from the hybridoma described in the present invention, thereby producing a dry analytical element (a reagent) that is similar to that as described above. Thus, there can be produced a single dry immunoanalytical reagent capable of quantifying both canine CRP and human CRP. By using this reagent, both the concentration of human CRP in human serum and the concentration of canine CRP in canine serum can be measured as with the aforementioned latex reagent. Thus, it is not necessary to prepare two types of reagents, separately.

That is to say, it is an object of the present invention to provide a monoclonal antibody recognizing both canine CRP and human CRP, a hybridoma producing the aforementioned monoclonal antibody, an immunoanalytical reagent for measuring canine CRP and human CRP using the aforementioned monoclonal antibody, a dry analytical element for measuring canine CRP and human CRP using the aforementioned monoclonal antibody, and a method for measuring CRP using the aforementioned monoclonal antibody.

The present inventors have conducted intensive studies directed towards achieving the aforementioned object. The inventors have first performed immunization of animals such as mice or rats, and have confirmed an increase in the antibody titer of serum with respect to the immunogen. Thereafter, the inventors have collected lymph cells or splenic cells, and they have then fused such cells with myeloma cells to produce hybridomas. Thereafter, the inventors have cultured the fused cells in a selective medium. When screening was performed using a culture supernatant of the proliferating hybridomas, the inventors have applied a method of sensitively reflecting the coupling constant of an antibody, in addition to a conventional qualitative screening method. As a result, the present inventors have succeeded in reliably selecting a hybridoma that produces a useful antibody. The measurement method of reflecting the coupling constant of an antibody is achieved by allowing an antibody and an antigen during the reaction to come into contact with an antigen protein or an antigen derivative in a concentration at the same level as, or higher or lower by an order of approximately 1 or 2 than the reciprocal of a target coupling constant (for example, when such target coupling constant is 10⁹M⁻¹, each antigen protein or an antigen derivative of approximately 10⁻¹⁰ M to 10⁻⁶ M, and more preferably of approximately 10⁻⁹M~10⁻⁷M is contacted), and by combining this method with a measurement method having sensitivity sufficient therefor. The coupling constant of an antibody produced by the selected hybridoma can be estimated by comparing with the signal strength of an antibody having a known coupling constant under the same conditions. As expected, the thus obtained antibody, which is derived from a canine CRP/human CRP antibody-producing hybridoma, has high affinity for the two types of antigens. Using this antibody, a dry analytical element used in the measurement of canine CRP/human CRP could be prepared. The present invention has been completed based on these findings.

The present disclosure provides a monoclonal antibody or a fragment thereof, which recognizes canine CRP and human CRP, which has a coupling constant of 10⁷ M⁻¹ to 10⁹ M⁻¹ with respect to canine CRP and human CRP, and the subclass of which is IgG1,

The present invention provides a monoclonal antibody or a fragment thereof, which is produced by a hybridoma having accession No. FERM BP-11132.

The present invention further provides a hybridoma having accession No. FERM BP-11132.

The present invention further provides an immunoanalytical reagent used in the measurement of canine CRP and human CRP, which comprises the monoclonal antibody or fragment thereof of the present invention as mentioned above.

The present invention further provides an immunoanalytical reagent used in the measurement of canine CRP and human CRP, which comprises a latex reagent labeled with the monoclonal antibody or fragment thereof of the present invention as mentioned above.

The present invention further provides an immunoanalytical reagent used in the measurement of canine CRP and human CRP, which comprises the monoclonal antibody or fragment thereof of the present invention as mentioned above that is labeled with an enzyme.

Preferably, the enzyme is *Bacillus subtilis* α-amylase.

The present invention further provides a dry analytical element used in the measurement of canine CRP and human CRP, which comprises the monoclonal antibody or fragment thereof of the present invention as mentioned above that is labeled with an enzyme.

Preferably, the enzyme is *Bacillus subtilis* α-amylase.

The present invention further provides a method for measuring CRP contained in a sample, which comprises allowing a sample to come into contact with the immunoanalytical reagent used in the measurement of canine CRP and human CRP of the present invention as mentioned above or the dry analytical element used in the measurement of canine CRP and human CRP of the present invention as mentioned above.

In a microplate method, which has been widely used as a method of selecting an antibody-producing hybridoma to date, an excessive amount of antigen (substantially, an antigen concentration) is immobilized on a microplate in order to highly efficiently capture an antibody existing in a trace amount in a culture supernatant, and thus the antigen captures even an antibody having a relatively low coupling constant. Thus, in this method, antibodies are captured regardless of their coupling constant. Accordingly, an antibody produced by the obtained hybridoma is not always suitable for purposes. In general, an antibody is produced from a hybridoma obtained after cloning by a culture method or a mouse ascites method. Thereafter, the produced antibody is purified, and the coupling constant thereof is finally measured. If the antibody had had an inappropriate coupling constant, then all the operations should have been carried out again.

In contrast, a screening method is used in which the reactivity of an antibody with an antigen of interest can be confirmed even if the concentration of the antibody in the culture supernatant of a hybridoma is relatively low, so as to succeed in accurately selecting a hybridoma producing an antibody having a high coupling constant that meets purposes. That is to say, the hybridoma of the present invention is able to produce an antibody, which is selected by determining the properties of an antibody effective and necessary for the purpose of quantifying canine CRP and human CRP, such as the type of antibody (isotype/subclass), antigenic specificity, and a target coupling constant value, which recognizes both canine CRP and human CRP, and which has high affinity for the two types of antigens. Using an antibody derived from this hybridoma, an immunoassay reagent that enables the measurement of canine CRP and human CRP with a single reagent can be produced. As described above, by combining this method with evaluation of the necessary properties of an antibody, a highly useful antibody-producing hybridoma can be accurately selected, thereby reducing the number of cloning that require man-hours. As a result, a useful antibody can be obtained at low costs.

### Brief Description of the Drawings

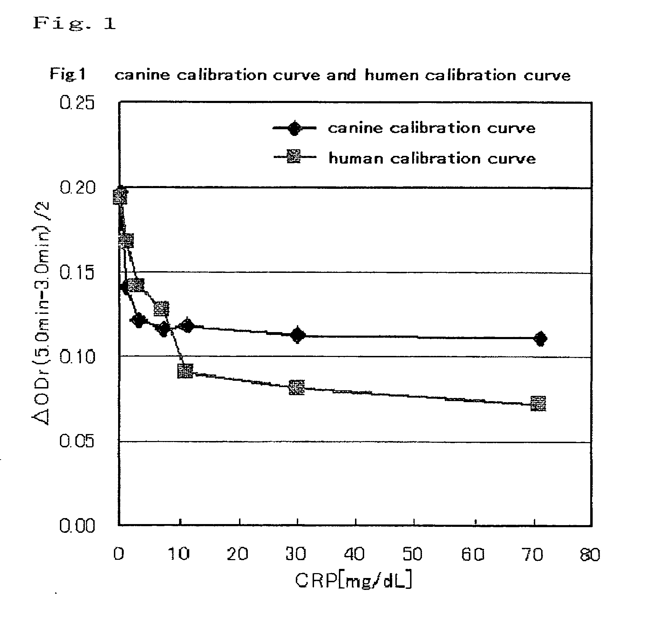

Figure 1 shows the results obtained by measuring CRP in human serum and canine serum using the antibody of the present invention.

### Best Mode for Carrying Out the Invention

The present invention will be more specifically described below.

The present invention relates to a monoclonal antibody, which recognizes canine CRP and human CRP, which has a coupling constant of 10⁷ M⁻¹ to 10⁹ M⁻¹ with respect to canine CRP and human CRP, and the subclass of which is IgG1. It is a monoclonal antibody produced by a hybridoma having accession No. FERM BP-11132. The term "antibody" is used in the present invention to mean not only an antibody molecule as a whole, but also a fragment thereof (for example, Fab, F(ab')₂, and Fab' fragments).

A monoclonal antibody can be obtained by an ordinary method. Specifically, an antigen is injected into the abdominal cavity or the like several times together with an adjuvant, and splenic cells are then collected. Thereafter, using polyethylene glycol or the like, the collected cells are fused with mouse myeloma cells. Thereafter, antibody-producing cells are cloned from the fused cells, and they are then allowed to proliferate as monoclone cells. The proliferating cells are further injected into the abdominal cavity of a mouse to obtain ascites and serum that contain a monoclonal antibody. More specifically, a monoclonal antibody can be obtained as follows.

First, CRP (canine CRP, etc.) is used as an antigen, and it is administered to a mammal such as a rat, a mouse, or a rabbit. The dose of the antigen per animal is 0.1 to 100 mg without the use of an adjuvant. It is 1 to 2000 µg with the use of an adjuvant. Examples of such adjuvant include a Freund's complete adjuvant (FCA), a Freund's incomplete adjuvant (FIA), and an aluminum hydroxide adjuvant. Immunization is mainly carried out by injecting an antigen into the vein, subcutis, or abdominal cavity. The immunization interval is not particularly limited. Immunization is carried out at intervals of several days to several weeks, and preferably 2 to 5 weeks, 1 to 10 times, and preferably 2 to 5 times. Thereafter, antibody-producing cells are collected 1 to 60 days, and preferably 1 to 14 days after the final immunization. Such antibody-producing cells include spleen cells, lymph node cells, peripheral blood cells, and others. Spleen cells or regional lymph node cells are preferable.

To obtain cell fusion hybridomas, cell fusion is carried out by fusing antibody-producing cells with myeloma cells. As myeloma cells to be fused with antibody-producing cells, generally available cell lines established from animals such as mice can be used. Used cell lines are preferably those, which do not survive in a HAT selective medium (containing hypoxanthine, aminopterin, and thymidine) in the state of unfused cells, and which survive only in a state in which they are fused with antibody-producing cells. Myeloma cells include mouse myeloma cell lines such as P3X63-Ag.8.U1(P3U1) and NS-I.

Subsequently, the aforementioned myeloma cells and antibody-producing cells are fused. In cell fusion, antibody-producing cells (1 ×10⁶ to 1 × 1.0⁷ cells/ml) are mixed with myeloma cells (2 × 10⁵ to 2 × 10⁶ cells/ml) in an animal cell culture medium containing no serum, such as DMEM or RPMI-1640 medium (the cell ratio between the antibody-producing cells and the myeloma cells is preferably 5:1), and a fusion reaction is carried out in the presence of a cell fusion promoter. As such cell fusion promoter, polyethylene glycol having a mean molecular weight of 1000 to 6000 daltons or the like can be used. Moreover, antibody-producing cells can also be fused with myeloma cells using a commercially available cell fusion apparatus that utilizes electrical stimulation (for example, electroporation).

Hybridomas of interest are selected from the cells that have undergone cell fusion. As a selection method, a cell suspension is diluted, as appropriate, with a fetal bovine serum-containing RPMI-1640 medium or the like, and it is then dispersed on a microtiter plate in a concentration of approximately 3 × 10⁵ cells/well. Thereafter, a selective medium is added to each well, and a culture is then carried out while appropriately exchanging the medium with a fresh one. As a result, cells that grow approximately 14 days after initiation of the culture in the selection medium can be obtained as hybridomas.

Subsequently, screening is carried out to examine the presence or absence of an antibody of interest in a culture supernatant of such growing hybridomas. The screening of hybridomas may be carried out by an ordinary method. Thus, the type of the screening method is not particularly limited. For example, an aliquot of a culture supernatant contained in a well in which hybridomas have grown may be collected, and it may be then screened by an enzyme immunoassay (ELISA, etc.), a radioimmunoassay, or the like. In the present invention, screening is carried out by a method of sensitively reflecting the coupling constant of an antibody, so as to reliably select hybridomas that produce useful antibodies. Such method of reflecting the coupling constant of an antibody can be carried out by allowing an antibody and an antigen during the reaction to come into contact with an antigen protein or an antigen derivative in a concentration at the same level as, or higher or lower by an order of approximately 1 or 2 than the reciprocal of a target coupling constant (for example, when such target coupling constant is 10⁹ M⁻¹, each antigen protein or an antigen derivative of approximately 10⁻¹⁰M to 10⁻⁶M, and more preferably of approximately 10⁻⁹M~10⁻⁷M is contacted), and by combining this method with a measurement method having sensitivity sufficient therefor.

The cloning of the fused cells is carried out by a limiting dilution method or the like, and hybridomas can be finally established as monoclonal antibody-producing cells.

As a method of collecting monoclonal antibodies from the established hybridomas, a common cell culture method, an ascites formation method, and the like can be adopted. In the cell culture method, hybridomas are cultured in an animal cell culture medium such as a 10% fetal bovine serum-containing RPMI-1640 medium, an MEM medium, or a serum free medium, under general culture conditions (for example, 37°C, 5% CO₂ concentration) for 7 to 14 days. Thereafter, antibodies are obtained from a culture supernatant.

In the case of the ascites formation method, approximately 1 × 10⁷ hybridomas are administered into the abdominal cavity of an animal of the same species as an mammal from which myeloma cells are derived, so that large quantities of hybridomas are allowed to proliferate therein. One or two weeks later, ascites is collected. When it is necessary to purify antibodies in the aforementioned method of collecting antibodies, the collected antibodies can be purified by appropriately selecting a method from known purification methods such as ammonium sulfate fractionation, ion exchange chromatography, gel filtration, and affinity chromatography (protein A-aragose, etc.), or by applying these purification methods in combination.

Examples of an antibody subclass include IgG1, GgG2a, and IgG2b. In the present invention, there is used IgG1 exhibiting good antibody fragmentation efficiency when an enzyme-antibody complex is produced. The obtained IgG1 may be converted to F(ab')₂ by eliminating its Fc portion with protease such as activated papain, pepsin, etc. The F(ab')₂ may be further reduced, so that it can be induce to a Fab' fragment.

In a preferred embodiment, a Fab' fragment is used as a monoclonal antibody. An intact antibody (IgG) has Fab (an antigen-binding site) and Fc (a complement-binding site). When an intact antibody is allowed to bind to an enzyme, and the obtained product is used as an enzyme-labeled antibody, if a sample is a blood sample, a complement component in the blood may bind to an Fc portion, thereby causing steric hindrance and inhibiting enzyme activity. Even in a case in which a sample is not a blood sample, since such Fc portions are non specifically adsorbed on the pores of a porous member or the surfaces of internal voids that constitute the wall or immune reaction layer of a reactor, the activity of an enzyme-labeled antibody becomes apparently low, and this causes noise during the measurement. To eliminate such noise, it is desired to use an Fab', F(ab')₂ or Fab fragment which does not contain Fc portion, as an antibody. Of these, an Fab' fragment having a free SH group is most preferably used as an antibody, in terms of convenience for binding to enzyme.

The aforementioned monoclonal antibody of the present invention, a latex reagent labeled with the aforementioned monoclonal antibody or a fragment thereof, and the aforementioned monoclonal antibody or a fragment thereof labeled with enzyme (for example, *Bacillus sublilis* α-amylase, etc.) can be used as an immunoanalytical reagent for measuring canine CRP and human CRP.

Enzyme used as a labeling substance can be selected while taking into account a combination of the enzyme with an enzyme substrate used in the subsequent enzyme reaction. In the present invention, reactivity with an enzyme reacting with an enzyme substrate is suppressed by steric hindrance caused by formation of a matrix-like structure consisting of an enzyme, an antibody, and an antigen. Thus, as a combination of an enzyme with a substrate, a system for easily detecting the influence of such steric hindrance is preferably selected. That is to say, in terms of sensitivity, a relatively high-molecular-weight enzyme substrate is preferable. For example, a substrate with a molecular weight of approximately 20,000 or more, and more preferably of approximately 100,000 or more, is used. Such substrate for amylase is starch; such substrate for cellulase is cellulose; such substrates for protease are proteins such as gelatin or hemacyanin; and such substrates for lipase are various types of lipids. JP Patent Publication (Kokai) Nos. 60-108756 A (1985), 60-171461 A (1985), and 60-171460 A (1985) disclose in detail selection of the aforementioned enzyme and substrate. Among the aforementioned combinations of enzymes with substrates, amylase (starch is an enzyme substrate) is preferable. In addition, water-insoluble substrates are particularly preferably used because the steric hindrance caused by a matrix-like structure consisting of an enzyme, an antibody, and an antigen is prominently manifested.

Examples of amylase include α-amylase, β-amylase, and glucoamylase. Amylase that is not substantially contained in a sample is preferable in terms of noise prevention. The origin of such amylase varies widely from animals (saliva, pancreatic juice, etc.) and plants to microorganisms. Thus, when the body fluid, blood, or the like of a human or animal is analyzed, it is preferable not to use amylase derived from such higher animal.

Examples of amylases derived from microorganisms or plants include: glucoarnylases derived from Aspergillus sp., Rhizopus sp., Saccharomyces sp., etc.; β-amylases derived from barley malt, wheat, soybean, etc.; and α-amylases derived from *Bacillus Subtilis, Streptomyces griseus, Pseudomonas stutzeri, Thermoactiamyces vulgaris,* etc. Of these, α-amylase derived from *Bacillus Subtilis* is most preferable because it has high liquefying power and excellent heat stability.

These enzymes are preferably not affected by interfering factors existing in all analytes. In addition, it is preferable that no competitive enzymes of the same species be present in an analyte. If an enzyme of the sample species as a labeling enzyme were contained in an analyte, an enzyme inhibitor might be used. The level of such enzyme inhibitor to inhibit an enzyme contained in an analyte may be greater than the level thereof to inhibit the activity of a labeling enzyme. An enzyme inhibitor that completely deactivates an enzyme contained in an analyte but that does not inhibit at all a labeling enzyme is most preferable. From a practical viewpoint, however, it is sufficient if a blank value is not increased by such enzyme inhibitor during the measurement. Thus, it is acceptable even if an enzyme inhibitor is deactivated and the activity of enzyme contained in a sample is recovered after the measurement. The type of such enzyme inhibitor is not particularly limited, as long as it does not inhibit the enzyme of an enzyme-labeled antibody. It is acceptable even if such enzyme inhibitor inhibits an enzyme in a free state. As such enzyme inhibitor, that having the aforementioned specificity may be selected from known enzyme inhibitors and may be used. Otherwise, an antibody reacting with problematic enzyme contained in an analyte may be produced, and it may be used as an enzyme inhibitor.

When α-amylase is used as an enzyme, carboxymethylated starch, starch, amylose, amylopectin, or the like may be used as a substrate. In particular, if water-insoluble starch or the like is used, an enzyme reaction takes place on the surface of a substrate particle, namely, on the interface between solid and liquid. As a result, the influence of steric hindrance due to an antibody-antigen bond upon enzyme activity appears significantly. Thus, the use of such water-insoluble starch is preferable in terms of sensitivity. Moreover, water-insoluble dye starch may be used to detect dye (pigment) adhering to soluble amylose as an enzyme decomposed product. As such water-insoluble blue starch polymer, a commercially available product such as Neo-Amylase (manufactured by Daiichi Kagaku Yakuhin Co.) can be used.

A method of binding an enzyme with an antibody can be carried out utilizing the functional groups of the two substances (an amino group, a carboxyl group, a thiol group, etc.). Representative binding methods include a glutaraldehyde method, a periodic acid method, a pyridyl disulfide method, and a maleimide-succinimide method. However, examples of such binding method are not limited thereto. In addition to the aforementioned methods, a method may be selected, as appropriate, from those described in publications such as "Method in Immunology and Immunochemistry," Vol. 1, C. A. Williams, M. W. Chase, Academic Press, 1967, or "Koso Meneki Sokutei Ho (Enzyme Immunoassay)" edited by Ishikawa, Kawai, and Miyai, Igaku-Shoin, Ltd., 1978, and it may be then used. Among these binding methods, a maleimide-succinimide method comprising binding a thiol group of an antibody hinge portion to an amino group of enzyme via crosslinkage is superior in terms of good reaction efficiency and ability to maintain antibody activity.

In such maleimide-succinimide method, an enzyme is allowed to bind to Fab', for example, as follows. First, an amino group of the enzyme is maleimidated with a maleimide-succinimide reagent. The resultant is purified by gel filtration, and it is then combined with an antibody having a thiol group (Fab'). In this complex formation, two or more types of antibodies having different epitopes (Fab') may be used in combination. In such a case, such antibody fragments are also subjected to a binding reaction. This complex formation reaction is preferably carried out using 3 to 7 moles of antibodies with respect to 1 mole of enzyme. When Fab' (molecular weight:
approximately 50,000) is used as an antibody and α-amylase (molecular weight: 50,000) is used as an enzyme, a binding reaction is preferably carried out using 1/3 to 1/7 by weight of α-amylase with respect to the total weight of Fab'. This binding reaction is generally carried out at 4°C to room temperature.

The generated enzyme-antibody complex (enzyme-labeled antibody) is purified by gel filtration, and as necessary, it is then dried (for example, by freeze-drying). The binding ratio between an enzyme and each antibody is not limited to 1 : 1, and any given ratio can be applied depending on purposes. Since enzyme generally has a large number of amino groups, multiple maleimide groups are introduced, and multiple antibody molecules are introduced into a single enzyme molecule. Since at least one antibody molecule should bind to one enzyme molecule, the molar ratio of such antibody to such enzyme in a complex should be 1 or greater. In order to reliably enhance detection sensitivity, such molar ratio is preferably set between 2 and 5. When Fab' (molecular weight: approximately 50,000) is used as an antibody and α-amylase (molecular weight: 50,000) is used as am enzyme, from the viewpoint of high detection sensitivity, the molecular weight of a complex is 150,000 daltons or more, and preferably 250,000 to 300,000 daltons.

Next, a method for measuring canine CRP and human CRP (a wet process) will be described. First, an antigen contained in an analyte is allowed to come into contact with an enzyme-antibody complex in a solution. During this process, it is adequate that the temperature of the solution be set between approximately 20°C and 45°C, and that pH be generally set between approximately pH 4.0 and 8.5. In order to keep pH constant, a buffer solution such as a phosphate buffer or an acetate buffer may be used, as necessary. The time necessary for contacting an antigen, with an enzyme-antibody complex is not particularly limited, as long as it is sufficient for the reaction of the two components. When the temperature of the solution is 37°C for example, an adequate reaction time is 20 to 30 minutes. Thereafter, an enzyme substrate is added to the reaction system, and the enzyme activity of the enzyme-antibody complex is then measured. If a test antigen is present in the analyte, it can be detected in the form of suppression of the enzyme activity. If a calibration curve has previously been plotted with a solution containing a known amount of test antigen, the amount of the test antigen contained in the analyte can be quantified.

It may also be possible to carry out only the reaction of an antigen with an enzyme-antibody complex in a solution system, and to subject a reaction solution obtained after the reaction to a dry analysis. Specifically, a dry analytical element comprising a substrate layer containing an enzyme substrate of a labeling enzyme may be prepared, and a reaction solution obtained after an immune reaction may be added to the element, so as to measure enzyme activity. As such layer structure, there can be used a dry analytical element which will be described below, from which an immune reaction layer has been eliminated.

Next, a dry analytical element comprising the monoclonal antibody of the present invention will be described. Specific examples of the configuration of such dry analytical element include those shown in Figures 1 and 2 of Japanese Patent No. 3151080.

That is to say, as an example, a detection layer (or a reagent layer) and an immune reaction layer are laminated on a light-transmittable support. The immune reaction layer is composed of a water-penetrable layer, and it contains the enzyme-labeled antibody of the present invention and a non-diffusible substrate used as a substrate of a labeling enzyme. The reagent layer is composed of a water-penetrable layer, and it contains a reagent composition for detecting an enzyme reaction product (a diffusible substance) dispersed and moved from the immune reaction layer. When such enzyme reaction product is a substance that can be directly detected, such as a colored substance, it is not necessary for the detection layer (or reagent layer) to contain a reagent composition used in detection. In this case, the detection layer (or reagent layer) functions as a detection layer.

An analyte (an antigen) contained in a liquid sample which was supplied to the element is bound to an enzyme-labeled antibody via an antigen-antibody reaction in the immune reaction layer so as to form a matrix structure. Thus, enzyme activity on a substrate contained in the same immune reaction layer is suppressed. As a result, the amount of the antigen contained in the analyte can be measured based on the amount of an enzyme reaction product detected in the reagent layer (or detection layer).

Moreover, as another example, an enzyme-labeled antibody and an enzyme substrate may be added to different layers. In this case, a water-penetrable substrate layer containing an enzyme substrate is disposed on a reagent layer (or a detection layer), and an immune reaction layer containing an enzyme-labeled antibody is further disposed thereon. In this case, an analyte (an antigen) contained in a liquid sample which was supplied to the element is bound to the enzyme-labeled antibody via an antigen-antibody reaction in the immune reaction layer to form a matrix structure, so that the analyte becomes substantially immovable. An enzyme-labeled antibody that has not bound to the antigen (or a matrix structure that is small enough not to be captured by a layer structure) moves to the subsequent substrate layer.

In any one of the aforementioned embodiments, an enzyme immune reaction can be promoted in the element only by adding a liquid sample to the element.

Furthermore, the dry analytical element of the present invention may be prepared as a multilayer comprising an immune reaction layer (or an immune reaction layer and a substrate layer), a reagent layer (or a detection layer), a support, a spreading layer, a detection layer, a light shielding layer, an adhesion layer, a water absorption layer, an undercoating layer, and other layers. Such analytical elements are disclosed, for example, in the specifications of JP Patent Publication (Kokai) No. 49-53888 A (1974) (the corresponding US Patent No. 3,992,158), JP Patent Publication (Kokai) No. 51-40191 A (1976) (the corresponding US Patent No. 4,042,335), JP Patent Publication (Kokai) No. 55-164356 A (1980) (the corresponding US Patent No. 4,292,272), and JP Patent Publication (Kokai) No. 61-4959 A (1986) (the corresponding EPC Laid-Open Patent No. 0166365A).

When a light-transmittable water-impenetrable support is used, the dry immunoanalytical element of the present invention may practically have the following structures. However, the contents of the present invention are not limited to the following examples.
(1) A structure having a reagent layer on a support and further having an immune reaction layer thereon.
(2) A structure having a reagent layer, a substrate layer, and an immune reaction layer on a support in this order.
(3) A structure having a reagent layer, an adhesion layer, and an immune reaction layer on a support in this order.
(4) A structure having a reagent layer, an adhesion layer, a substrate layer, and an immune reaction layer on a support in this order.
(5) A structure having a detection layer, a reagent layer, and an immune reaction layer on a support in this order.
(6) A structure having a detection layer, a reagent layer, a substrate layer, and an immune reaction layer on a support in this order.
(7) A structure having a reagent layer, a light reflection layer, and an immune reaction layer on a supporting this order.
(8) A structure having a reagent layer, a light reflection layer, a substrate layer, and an immune reaction layer on a support in this order.
(9) A structure having a detection layer, a reagent layer, a light reflection layer, and an immune reaction layer on a support in this order.
(10) A structure having a detection layer, a reagent layer, a light reflection layer, a substrate layer, and an immune reaction layer on a support in this order.
(11) A structure having a detection layer, a light reflection layer, a reagent layer, and an immune reaction layer on a support in this order.
(12) A structure having a detection layer, a light reflection layer, a reagent layer, a substrate layer, and an immune reaction layer on a support in this order.
(13) A structure having a second reagent layer, a light reflection layer, a first reagent layer, and an immune reaction layer on a support in this order.
(14) A structure having a second reagent layer, a light reflection layer, a first reagent layer, a substrate layer, and an immune reaction layer on a support in this order.
(15) A structure having a detection layer, a second reagent layer, a light reflection layer, a first reagent layer, and an immune reaction layer on a support in this order.
(16) A structure having a detection layer, a second reagent layer, a light reflection layer, a first reagent layer, a substrate layer, and an immune reaction layer on a support in this order.

In the structures described in (1) to (12) above, the reagent layer may consist of multiple different layers. A water absorption layer may be established between a support and a reagent layer or a detection layer. In addition, a filtration layer may be established between individual layers. Moreover, a spreading layer may be established on a substrate layer, or developing action may be imparted to the substrate layer so that the layer can function as a spreading layer. When a solid component such as a hemocyte is contained in an analyte, a suitable filtration layer may be established as the uppermost layer of the analytical element.

Either an immune reaction layer or a substrate layer is composed of a water penetrable layer. To secure the water penetrability of such layer, such layer is preferably prepared as a porous layer of a porous medium or as a layer of a hydrophilic polymer binder.

Such porous layer may be either fibrous or non-fibrous. As a fibrous material, a filter, a non-woven fabric, a woven fabric (e.g. a plain-woven fabric), a knitted fabric (e.g. a tricot knitted fabric), a glass fiber filter, etc. can be used, for example. As a non-fibrous material, a membrane filter of cellulose acetate or the like described in JP Patent Publication (Kokai) No. 49-5388 (1974) A, a continuous void-containing granular structure layer of inorganic or organic fine particles described in JP Patent Publication (Kokai) No. 49-53888 (1974) A, JP Patent Publication (Kokai) No. 55-90859 (1980) A (the corresponding US Patent No. 4,258,001), and JP Patent Publication (Kokai) No. 58-70163 (1983) A (the corresponding US Patent No. 4,486,537), and the like may be used. A lamination layer product of multiple porous layers that are partially adhered to one another described in JP Patent Publication (Kokai) No. 61-4959 (1986) A (the corresponding European Paten Application Laid-Open No. EP 0166365A), JP Patent Publication (Kokai) No. 62-116258 (1987) A, JP Patent Publication (Kokai) No. 62-138756 (1987) A (the corresponding European Paten Application Laid-Open No. EP 0226465A), JP Patent Publication (Kokai) No. 62-138757 (1987) A (the corresponding European Paten Application Laid-Open No. EP 0226465A), JP Patent Publication (Kokai) No. 62-138758 (1987) A (the corresponding European Paten Application Laid-Open No. EP 0226465A), etc. is also preferably used.

The porous layer may be a spreading layer having what is called "measuring action" to develop liquid on an area that is almost proportional to the amount of the liquid to be supplied. As such spreading layer, a woven fabric, a knitted fabric, or the like is preferable from among the aforementioned examples. A glow discharge treatment as described in JP Patent Publication (Kokai) No. 57-66359 (1982) A may be performed on a woven fabric. In order to control a spreading area, a spreading rate, etc., a spreading layer may comprise a hydrophilic polymer or a surfactant as described in JP Patent Publication (Kokai) No. 60-222770 (1985) A (the corresponding EP 0162301A), JP Patent Publication (Kokai) No. 63-219397 (1988) A (the corresponding West German Patent Application Laid-Open No. DE 37 17 913A), JP Patent Publication (Kokai) No. 63-112999 (1988) A (the corresponding DE 37 17 913A), and JP Patent Publication (Kokai) No. 62-182652 (1987) A (the corresponding DE 37 17 913A).

An example of a useful method of adding a substrate to a porous layer is a method, which comprises: previously impregnating or coating a porous membrane or the like of a paper, a fabric or a polymer with a substrate; and adhering the porous membrane onto another water-penetrable layer such as a reagent layer established on a support according to the method described in JP Patent Publication (Kokai) No. 55-164356 (1980) A. There may be an alternative method, which comprises adhering a porous layer to another water-penetrable layer (e.g. a reagent layer) according to the aforementioned method, and then coating the porous layer with a composition containing a substrate. Such porous layer may be impregnated or coated with a composition containing a substrate by known methods. As such coating method, dipping coating, doctor coating, hopper coating, curtain coating, or the like may be selected, as appropriate, and may be then used. The thickness of the thus produced substrate layer is not particularly limited. When such substrate layer is established as a coating layer, the thickness of the layer is approximately 1 µm to 50 µm, and preferably 2 µm to 30 µm. When such substrate layer is produced by methods other than a coating method, such as lamination with a laminate, the thickness of the layer may vary largely from several tens of µm to several hundreds of µm.

When an immune reaction layer and a substrate layer are constituted with a water-penetrable layer of a hydrophilic polymer binder, the following materials may be used as hydrophilic polymers: gelatin and a derivative thereof (e.g. phthalic gelatin), a cellulose derivative (e.g. hydroxyethylcellulose), agarose, sodium alginate, an acrylamide copolymer, a methacrylamide copolymer, a copolymer of acrylamide or methacrylamide with various types of vinyl monomers, polyhydroxyethyl methacrylate, polyvinyl alcohol, polyvinylpyrrolidone, sodium polyacrylate, and a copolymer of acrylic acid with various types of vinyl monomers.

A substrate layer constituted with a hydrophilic polymer binder can be formed by coating a support or another layer such as a detection layer with an aqueous solution or a water dispersion containing a substrate, a reagent composition and a hydrophilic polymer according to the methods described in the specifications of JP Patent Publication (Kokoku) No. 53-21677 (1978) B (the corresponding US Patent No. 3,992,158), JP Patent Publication (Kokai) No. 55-164356 (1980) A (the corresponding US Patent No. 4,292,272), JP Patent Publication (Kokai) No. 54-101398 (1979) A (the corresponding US Patent No. 4,132,528), JP Patent Publication (Kokai) No. 61-292063 (1986) A (Chemical Abstracts, 106, 210567y), etc., and then drying it. The thickness of a substrate layer comprising a hydrophilic polymer as a binder in a dry state is between approximately 2 µm and 50 µm, and preferably between approximately 4 µm and 30 µm. The coating amount is between approximately 2 g/m²and 50 g/m², and preferably between approximately 4 g/m²and 30 g/m².

For the purpose of improving various properties such as coating property, the dispersibility of a diffusible compound, reactivity, and preserving property, a substrate layer may also comprise various types of organic or inorganic additives such as an enzyme activator, a coenzyme, a surfactant, a pH buffer composition, fine particles, and an antioxidant, in addition to a non-diffusible substrate. Examples of a buffer that may be contained in the substrate layer include pH buffer systems described in "Kagaku Binran Kiso Hen (Chemistry Handbook Basic Version)" edited by the Chemical Society of Japan, Tokyo, Mazuren Co., Ltd., 1966, pp. 1312-1320; "Data for Biochemical Research" edited by R. M. C. Dawson et al., 2nd edition, Oxford at the Clarendon Press, 1969, pp. 476-508; Biochemistry, 5, pp. 467-477, 1966; and Analytical Biochemistry, 104, pp. 300-310,1980. Specific examples of such pH buffer include: a buffer comprising Tris(hydroxymethyl)aminomethane (Tris); a buffer comprising phosphate; a buffer comprising borate; a buffer comprising citric acid or citrate; a buffer comprising glycine; a buffer comprising Bicine; a buffer comprising HEPES; and a Good's buffer such as a buffer comprising MES.

An immune reaction layer may have the same structure as that of the aforementioned substrate layer. In order to add a substrate and an enzyme-labeled antibody to a single layer or two layers adjacent to each other in a substantially dry state or in the substantially absence of water, such enzyme-labeled antibody may be dissolved or dispersed in a non-aqueous solvent such as alcohol (e.g. ethanol), and thereafter, a water-penetrable layer may be impregnated with the non-aqueous solvent.

A reagent layer comprises a reagent composition for detecting a diffusible substance dispersed and moved from the immune reaction layer (or the substrate layer). As necessary, such reagent composition comprises a depolymerizing enzyme, and comprises a detection reagent composition for detecting a low-molecular-weight product generated as a result of reduction in the molecular weight of a diffusible substance. The reagent layer is constituted with a water-penetrable layer. It is preferably a continuous layer consisting of a hydrophilic polymer binder, from among the water-penetrable layers described in the above substrate layer. The type of a hydrophilic polymer binder used is determined, while taking into consideration a diffusible product generated in the substrate layer or a coloring reagent contained in the reagent layer.

When a diffusible substance dispersed and moved from the immune reaction layer (or the substrate layer) can be directly detected, it is unnecessary to add a detection reagent composition to the reagent layer. The reagent layer functions as a detection layer. Even if the reagent layer functions as a detection layer, it is preferably a continuous layer consisting of a hydrophilic polymer binder, from among the water-penetrable layers described in the above substrate layer.

Any one of light non-transmittable (opaque), light semi-transmittable (semi-transparent), and light transmittable (transparent) supports can be used as a support. In general, a light transmittable water non-penetrable support is preferable. Preferred materials of such light transmittable water non-penetrable support include polyethylene terephthalate and polystyrene. In order to strongly adhere a hydrophilic layer to the support, in general, an undercoating layer is established, or a hydrophilic treatment is performed on the support.

As with the inventions described in JP Patent Publication (Kokai) No. 3-295466 (1991) A (the corresponding EP 0451848A), JP Patent Publication (Kokai) No. 4-128655 (1992) A, and JP Patent Publication (Kokai) No. 4-2765551 (1992) A, in the dry immunoanalytical element of the present invention, an increase in sensitivity can be achieved by adding to a reagent layer a depolymerizing enzyme that further depolymerizes a diffusible substance decomposed from a non-diffusible substrate by a labeled enzyme. A combination of a labeled enzyme, a non-diffusible substrate, a diffusible substance, and a depolymerizing enzyme, may be selected from combinations of these components, in which an enzyme acts on a non-diffusible substrate to generate a diffusible substance, and a depolymerizing enzyme as described later acts on the diffusible substance so as to convert it to a lower molecular weight product, which can be easily detected.

That is, as an antibody-labeling enzyme, there is selected an enzyme that decomposes a high-molecular-weight non-diffusible substrate to generate a diffusible product that is further converted to a lower molecular weight product by action of a depolymerizing enzyme. As a non-diffusible substrate, there is selected a substrate that is non-diffusible (insoluble) in an aqueous analyte solution and that is neither dispersed nor moved from an immune reaction layer (or a substrate layer) to a reagent layer. As a depolymerizing enzyme, there is selected an enzyme that depolymerizes a diffusible product generated from a non-diffusible substrate by the action of an antibody-labeling enzyme so as to convert it to a detectable low-molecular-weight product. Specific examples of such depolymerizing enzyme will be given below.

As such enzyme, a catabolic enzyme that produces a diffusible oligomer from a non-diffusible substrate consisting of a polymer is preferable. Among the aforementioned labeling enzymes, a saccharide-hydrolyzing enzyme is preferable, for example. Examples of such saccharide-hydrolyzing enzyme include α-amylase, β-amylase, glucoamylase, and lysozyme.

Examples of a substrate for the aforementioned α-amylase, β-amylase, and glucoamylase include carboxymethylated starch and starch. When such carboxymethylated starch or starch is used as a non-diffusible substrate, the following combination may be applied. That is, α-amylase is used as a labeling enzyme and glucoamylase or α-glucosidase as described below is used as a depolymerizing enzyme.

Such depolymerizing enzyme may be of the same type as that of a labeling enzyme. In this case, the labeling enzyme is an endo-active enzyme that cleaves the inside of a molecule to produce an oligomer. The polymerizing enzyme is preferably an enzyme having exo activity to act on the end of a molecule to produce a monomer. In a case in which a non-diffusible substrate is a polymer (e.g. starch) for example, an enzyme that is able to decompose a diffusible oligomer (e.g. maltose) produced by a labeling enzyme into a monomer (e.g. glucose) is used as a depolymerizing enzyme. An example of such depolymerizing enzyme is saccharide-hydrolyzing enzyme. More specifically, α-amylase, β-amylase, glucoamylase, and α-glucosidase may be used. When carboxyl methyl cellulose and cellulase are used as a non-diffusible substrate and a labeling enzyme, respectively, C1 enzyme can be used as a depolymerizing enzyme.

As a combination of such labeling enzyme, non-diffusible substrate, and depolymerizing enzyme, they can be selected from enzymes and substrates described in known publications (for example, "Koso Handbook (Enzyme Handbook)," Bunji Maruo, Nobuo Tamiya, Asakura Publishing Co., Ltd., 1982, and "Seikagaku Handbook (Biochemical Handbook)" Nobumasa Imura, et al., Maruzen Co., Ltd., 1984).

A low-molecular-weight product produced by depolymerizing enzyme in a reagent layer can be optically detected using a known detection system reagent. As a method of detecting glucose finally generated by the aforementioned depolymerizing enzyme, the following known methods may be applied: a method comprising oxidizing glucose in the presence of glucose oxidase and detecting the generated hydrogen peroxide (for example, a method using the following reagent: a Trinder reagent described in Ann. Clin. Biochem., 6, 24 (1964); a Trinder reagent described in J. Clin. Pathol., 22, 246 (1969); Trinder reagents described in JP Patent Publication (Kokai) No. 49-50991 (1974) A (the corresponding US Patent No. 3,886,045), US Patent No. 3,992,158, JP Patent Publication (Kokai) No. 55-164356 (1980) A (the corresponding US Patent No. 4,292,272), etc.; reagents comprising triaryl-substituted imidazole leuco dye described in JP Patent Publication (Kokai) No. 53-2618 (1978) A (the corresponding US Patent No. 4,089,747), JP Patent Publication (Kokai) No. 58-45557 (1983) A, etc.; or reagents comprising diaryl-monoaralkyl-substituted imidazole leuco dye described in JP Patent Publication (Kokai) No. 59-193352 (1984) A (the corresponding EP 0122641A), JP Patent Publication (Kokai) No. 60-224677 (1985) A (the corresponding US Patent No. 4,665,023), etc.); a method of detecting NADH generated in the presence of glucose dehydrogenase and NAD; and a method of detecting glucose-6-phosphate generated in the presence of hexokinase. Among these detection methods, a method comprising oxidizing glucose in the presence of glucose oxidase and detecting the generated hydrogen peroxide using peroxidase and leuco dye is most preferable in terms of sensitivity.

These detection reagents may be contained in the reagent layer of the analytical element together with a depolymerizing enzyme. However, it may also be possible to add such detection reagent to another layer (for example, the second reagent layer, the detection layer, etc.) established under the reagent layer so as to carry out detection in this layer. When leuco dye is used, such leuco dye is preferably dispersed in a hydrophilic polymer in a solution of a water-nonmiscible solvent from the viewpoint of the stability of the generated dye.

The dry immunoanalytical element of the present invention can be prepared by known methods described in the aforementioned patent specifications. From the viewpoint of production, wrapping, transportation, preservation, measurement operations, etc., it is preferable that the analytical element of the present invention be cut into a small piece such as a square with a side of approximately 15 mm to approximately 30 mm or a circle having almost the same above size, and that it be then placed into the slide frame described in JP Patent Publication (Kokoku) No. 57-28331 (1982) B (the corresponding US Patent No. 4,169,751), JP Utility Model Publication (Kokai) No. 56-142454 (1981) U (the corresponding US Patent No. 4,387,990), JP Patent Publication (Kokai) No. 57-63452 (1982) A, JP Utility Model Publication (Kokai) No. 58-32350 (1983) U, JP Patent Publication (Kohyo) No. 58-501144 (1983) A (the corresponding International Publication WO83/00391), or the like, so that it is used as a chemical analysis slide. Depending on intended use, the analytical element of the present invention may be processed into a long tape, which may be placed in a cassette or a magazine and may be then used. Otherwise, a small piece thereof may be attached to or placed in a card with an aperture and may be then used.

By carrying out the same operations as those described in the aforementioned patent specifications, the dry analytical element of the present invention can be used in the quantitative analysis of a high-molecular antigen that is a test substance contained in a liquid sample. For example, approximately 5 to 30 µL of, and preferably 8 to 15 µL of an aqueous liquid sample solution such as plasma, serum, or urine is added to a substrate layer. The resultant analytical element is incubated at a constant temperature between approximately 20°C and approximately 45°C, and preferably between approximately 30°C and approximately 40°C, for 1 to 10 minutes. Color development or discoloration occurring in the element is measured from the side of a light transmittable support via reflection photometry. Thereafter, the amount of the high-molecular antigen in the analyte can be obtained by the principle of colorimetry using the previously prepared calibration curve. The amount of a liquid sample added, the incubation time, and the temperature are kept constant, so that quantitative analysis can be carried out with high accuracy. Using chemical analysis apparatuses described in JP Patent Publication (Kokai) Nos. 60-125543 (1985) A, 60-220862 (1985) A, 61-294367 (1986) A, etc., highly accurate quantitative analysis can be carried out by extremely easy operations. Depending on purposes or required accuracy, the degree of color development may be determined by visual observation, and semi-quantitative measurement may be carried out. When the analytical element comprises no enzyme-labeled antibodies, an aqueous sample solution may be mixed with a solution containing an enzyme-labeled antibody before adding it to the element, so that a binding reaction may be sufficiently carried out. Thereafter, the reaction solution may be added to the substrate layer.

The present invention further relates to a method for measuring CRP in a sample, which comprises allowing a sample to come into contact with the aforementioned immunoanalytical reagent for measuring canine CRP and human CRP of the present invention, or the dry analytical element for measuring canine CRP and human CRP of the present invention. The type of a sample is not particularly limited. Examples of such sample include blood (whole blood, plasma, and serum), lymph, and urine. A preferred example is blood (whole blood, plasma, and serum), more preferred examples are serum and plasma, and a particularly preferred example is serum.

The present invention will be specifically described in the following examples. However, these examples are not intended to limit the scope of the present invention.

### Examples

### Example 1: Preparation of hybridoma that produces monoclonal antibody with IgG1 subclass having the same level of reactivity with canine CRP and human CRP

### (1) Preparation of antibody-producing fused cells (hybridomas):

Canine CRP separated and purified from the fresh serum of a beagle canine using an affinity column was used as an antigen. Mice (BALBc) were immunized with the antigen. After confirming that an antibody titer with respect to canine CRP and human CRP had increased, spleen cells were collected, and the collected spleen cells were then fused with myelomas (P3U1) by a PEG method. The fused cells were subjected to selective culture in an HAT medium, so as to obtain a large number of proliferating hybridomas.

### (2) Selection of hybridomas (1): Confirmation of antigenic specificity and confirmation of type and subclass of antibody

Each of the two types of protein antigens, canine CRP and human CRP, were diluted with PBS(-) so as to have a concentration of 1 µg/L, and were immobilized. An anti-mouse IgG HRP-labeled goat antibody (Goat anti-mouse IgG (H+L) HRP conjugated secondary antibody; No. AP308P; Funakoshi Corp.) was used as a secondary antibody. Thus, an ELISA method was carried out with a microplate so as to confirm the reactivity of the culture supernatant of the hybridomas obtained in (1) above against each antigen. As a result, several types of hybridomas producing antibodies reacting with either one or both of the two types of canine CRP and human CRP antigens could be selected. In addition, the isotypes and subclasses of antibodies in the culture supernant of these hybridomas were identified using commercially available identification kit reagents.

### (3) Selection of hybridomas (2): Confirmation of coupling constant to each antigen

With regard to culture supernatants which produced antibodies of IgG type selected from among culture supernatants whose reactivity with canine CRP and/or human CRP had been confirmed, the coupling constant was measured using the apparatus of Biacore (Biacore3000). Anti-mouse IgG rabbit IgG was immobilized on a sensor chip, and the sensor chip was then allowed to come into contact with the culture supernatant under certain conditions, so as to immobilize thereon mouse IgG existing in the culture supernatant. Subsequently, each antigen protein (4 µ/mL; 3,5 × 10⁻⁸ M) was allowed to come into contact with the culture supernatant (a target coupling constant: 10⁹M⁻¹). Using a signal increase occurring when the culture supernatant was allowed to come into contact (antibody signal: corresponding to the amount of IgG in the culture supernatant) as a baseline, a signal increase occurring when an antigen protein was allowed to come into contact (antigen signal: corresponding to the ability of an antibody to capture an antigen) was compared with the aforementioned signal increase as a baseline. Thus, the order of the coupling constants of antibodies contained in the culture supernatant can be assumed. The measurement results of the culture supernatant are shown in Table 1. It is to be noted that antibody FH-01 described in the lowermost case of Table 1 is an antibody with a coupling constant of 2 × 10⁸ M⁻¹ that has specificity only for human CRP. The estimated values of Kd are shown in Table 2. The ratio between antigen signal and antibody signal (hereinafter referred to as "signal ratio") in FH01 was 0.738, whereas the signal ratio of the antibody in the culture supernatant of hybridoma 16H4 was 0.887 with respect to canine CRP Thus, it was anticipated to have a coupling constant equivalent to or greater than that of FH01. In addition, the antibody of 16H4 exhibited a signal ratio of 0.234 with respect to human CRP under the same conditions. This value was slightly lower than that of FH01, but it was anticipated to have a sufficient coupling constant. Antibodies were prepared by a mouse ascites method using the three types of hybridomas, 3A6, 11C6, and 16H4, and the prepared antibodies were then purified with a protein A column. The signal ratios of the thus obtained antibodies under the same conditions were well matched with those of culture supernatants. These results demonstrate that the results obtained using culture supernatants are effective for selection of antibodies. By this method, hybridoma 16H4 could be selected as a hybridoma producing an antibody, which has a sufficient coupling constant (10⁷M⁻¹ to 10⁹M⁻¹) with respect to canine CRP and human CRP and whose subclass is IgG1. Moreover, as hybridomas producing antibodies reacting with only canine CRP, hybridomas 6A3 and 11 C6 could be obtained.

Hybridoma 16H4 (identification number assigned by depositor: MM44097-16H4F5) was deposited with the National Institute of Advanced Industrial Science and Technology, an Independent Administrative Institution under the Ministry of Economy, Trade and Industry, at the AIST Tsukuba Central 6, Higashi 1-1-1, Tsukuba, Ibaraki, Japan, under accession No. FERM P-21571 (reception No. FERM AP-21571) on April 18, 2008. The deposition of FERM P-21571 was transferred to the international deposition and was deposited under Budapest treaty under accession No. FERM BP-11132 on May 27, 2009.

**Table 1: Results of microplate ELISA and Biacore measurement of various types of hybridoma culture supernatant and purified clone culture supernatants**

| | | | | Biacore measurement *3 | | | |
|---|---|---|---|---|---|---|---|
| | | | | Antigen signal/antibody signal | | | |
| | | ELISA color development OD*2 | | Culture supernatant | | Purified clone antibody | |
| Hybridoma name | Isotype/subclass | Canine CRP | Human CRP | Canine CRP | Human CRP | Canine CRP | Human CRP |
| 5B8 | IgG2b | 2.752 | 0.080 | - | - | 0 | 0 |
| 8B5 | IgG2b | 2.955 | 3.027 | - | - | 0 | 0 |
| 8G11*1 | IgG1 | 1.487 | 2.474 | 0 | 0.075 | - | - |
| 3A6 | TgG1 | 0.111 | 0.091 | 0.368 | 0.027 | 0.555 | 0 |
| 11C6 | IgG1 | 0.300 | 0.085 | 0.535 | 0 | 0.502 | 0 |
| 16H4 | IgG1 | 0.706 | 1.633 | 0.887 | 0.234 | 0.882 | 0.230 |
| FH01*4 | IgG1 | - | - | - | - | 0 | 0.738 |

| | | | | | | | |
|---|---|---|---|---|---|---|---|
| *1 : Eliminated during cloning process *2: Antigen concentration during immobilization: 1 µg/mL *3: Antigen concentration: 4 µg/mL (approximately 3,5 × 10⁻⁸M) *4: Coupling constant with human CRP: 2 × 10⁸M⁻¹ | | | | | | | |

**Table 2**

| | 50% bound antigen concentration (M) | | Kd (M⁻¹) | |
|---|---|---|---|---|
| | Canine CRP | Human CRP | Canine CRP | Human CRP |
| FH01 | - | 5.76 × 10⁻⁹ | - | 1.74 × 10⁸ |
| FH02 | - | 3.98 × 10⁻⁹ | - | 2.51 × 10⁸ |
| 16H4 | 4.57 × 10⁻⁹ | 7.94 × 10⁻⁸ | 2.19 × 10⁸ | 1.26 × 10⁷ |

| | | | | |
|---|---|---|---|---|
| FH02 indicates another anti-human CRP monoclonal antibody. | | | | |

### Example 2: Preparation of monoclonal antibody

Hybridoma 16H4 was melted with RPMI 1640 + 10% FBS, and it was then cultured. Thereafter, 0.5 mL (5 × 10⁶ cells/mouse) of the thus proliferating cells was implanted into each of 20 mice. After a certain period of time, ascites was collected from the abdominal cavity and was then centrifuged. The supernatant was purified by the following protein A-affinity chromatography method. The supernatant of ascites diluted with 1.5 M glycine-NaOH (pH 8.9)-3 M NaCl was supplied to a protein A column equilibrated with the same above buffer, so that IgG was absorbed thereon. The adsorbed IgG was eluted with an elution buffer (100 mM citrate buffer; pH 3.0), and a neutralization solution (2 M Tris-HCl; pH 9.0) was immediately added thereto, so that the pH was adjusted to pH 7.0 to 7.5. The resultant was dialyzed against PBS, so as to obtain a purified antibody.

### Example 3: Preparation of enzyme-labeled antibody reagent

Applying a method similar to the method described in Japanese Patent No. 315080, anti-canine/human CRP/IgG Fab' was prepared from hybridoma 16H4, using *Bacillus subtilis* α -amylase.

### Example 4: Preparation of analytical element used in canine/human CRP measurement

A 180-µm colorless, transparent smooth film of polyethylene terephthalate undercoated with gelatin was coated with an aqueous solution of the following composition and dried to a thickness of 14 µm.

| | |
|---|---|
| Gelatin | 14.1 g/m² |
| Peroxidase | 12.0 KU/m² |
| Glucose oxidase | 6.0 KU/m² |
| Glucoamylase | 5.0 KU/m² |
| Leuco Dye | 0.5 g/m² |
| Surfactant | 1.0 g/m² |

As a surfactant, polyoxy(2-hydroxy)propylene nonylphenyl ether (Surfactant 10G; manufactured by Olin) was used, As a leuco dye, 2-(3,5-dimethoxy-4-hydroxyphenyl)-4-(4-dimethylaminophenyl)-5-phenethylimidazole acetate was used.

Subsequently, the aforementioned film was coated with an aqueous solution of the following composition and dried to a thickness of 10 µm.

| | |
|---|---|
| Gelatin | 10.2 g/m² |
| Surfactant | 0.5 g/m² |

Subsequently, the aforementioned film was coated with an aqueous solution of the following composition, whose pH had been adjusted to pH 6.4, and it was then dried to a thickness of 8 µm.

| | |
|---|---|
| Hydroxypropylcellulose | 4.7 g/m² |
| Carboxymethyl starch | 3.5 g/m² |
| PIPES | 0.9 g/m² |
| Mannitol | 2.3 g/m² |
| Surfactant | 1.2 g/m² |

Thereafter, water was supplied in an amount of approximately 60 g/m² onto the entire surface of the aforementioned film so as to wet the film. A tricot knitted fabric knitted from 50 deniers polyethylene terephthalate spun yarn by 36 gauges was laminated on the film by light compression, and it was then dried.

The aforementioned fabric was coated with ethanol to a concentration of 200 g/m² (= OC1 coating), and it was then dried. Thereafter, the resultant fabric was further coated with an ethanol solution of the following composition so as to obtain the following amounts of ingredients and to have a thickness after drying of 5 µm (= OC2 coating). The fabric was dried, so as to produce an integrated multilayer analytical element.

| | |
|---|---|
| Amylase-labeled anti-C-reactive protein mouse antibody | 14.0 KU/m² |
| Anti-C-reactive protein mouse second antibody | 6.2 mg/m² |
| Polyvinylpyrrolidone | 5.6 g/m² |
| Surfactant | 0.2 g/m² |

The aforementioned integrated multilayer analytical element was cut into a chip of 12 mm x 13 mm, and the chip was then placed into a slide frame (described in JP Patent Publication (Kokai) No. 57-63452 (1982) A, so as to produce a dry slide used in CRP analysis according to the present invention.

### Example 5: Performance evaluation test

A diluent of the following composition (*) and a liquid prepared by 21 times diluting human serum (CRP concentrations: 0, 1.1, 3.0, 7.1, 11, 30, and 71 mg/dL; detection by immunonephelometry) were added in an amount of 10 µL to the dry slide used in CRP analysis prepared in the above-described example.

Thereafter, while incubating at 37°C for 5 minute, the reflection density at 650 nm was measured by Fuji Dri-Chem 5000 (manufactured by Fuji Photofilm Co., Ltd.) every 10 seconds. The reflection density (ΔODr) per minute was obtained based on the reflection densities from 3 to 5 minutes during the aforementioned measurement. The same measurement was also performed on canine serum. The results are shown in Table 1 and Figure 1.

### * Composition of diluent

| | |
|---|---|
| MES*1 | 5 mg |
| Casein aqueous solution *2 | 100mg |
| Sodium azide | 0.2mg |
| Purified water | 1.0ml |

| | |
|---|---|
| *1 MES: 2-(N-morpholino)ethanesulfonic acid monohydrate *2 e.g. Product name: Block Ace | |

**Table 1:**

| | Human scrum | Canine serum |
|---|---|---|
| CRP 0.0 mg/dL (diluent) | 0.193 | 0.197 |
| CRP 1.1 mg/dL | 0.167 | 0.141 |
| CRP 3.0 mg/dL | 0.141 | 0.121 |
| CRP 7.1 mg/dL | 0.127 | 0.116 |
| CRP 11 mg/dL | 0.091 | 0.118 |
| CRP 30 mg/dL | 0.081 | 0.113 |
| CRP 71 mg/dL | 0.072 | 0.111 |

The aforementioned results demonstrated that a change in ΔODr (reflection optical density) was observed corresponding to a density change up to CRP = 71 mg/dL in the case of human serum, and that such a change in ΔODr was observed corresponding to a density change up to CRP = 7.1 mg/dL in the case of canine serum. In the case of canine serum, it is possible to produce a calibration curve up to a high density of CRP by further increasing a dilution magnification.

## Claims

1. A monoclonal antibody or a fragment thereof, which is produced by a hybridoma having accession No. FERM BP-11132.

2. A hybridoma having accession No. FERM BP-11132.

3. An immunoanalytical reagent used in the measurement of canine CRP and human CRP, which comprises the monoclonal antibody or fragment thereof of claim 1.

4. An immunoanalytical reagent used in the measurement of canine CRP and human CRP, which comprises a latex reagent labelled with the monoclonal antibody or fragment thereof of claim 1.

5. An immunoanalytical reagent used in the measurement of canine CRP and human CRP, which comprises the monoclonal antibody or fragment thereof of claim 1 that is labelled with an enzyme.

6. An immunoanalytical reagent used in the measurement of canine CRP and human CRP according to claim 5, wherein the enzyme is Bacillus subtilis α-amylase.

7. A dry analytical element used in the measurement of canine CRP and human CRP, which comprises the monoclonal antibody or fragment thereof of claim 1 that is labelled with an enzyme.

8. The dry analytical element used in the measurement of canine CRP and human CRP according to claim 7, wherein the enzyme is Bacillus subtilis α-amylase.

9. A method for measuring CRP contained in a sample, which comprises allowing a sample to come into contact with the immunoanalytical reagent used in the measurement of canine CRP and human CRP of any of claims 3 to 6 or the dry analytical element used in the measurement of canine CRP and human CRP of claim 7 and 8.

## Patentansprüche

1. Monoklonaler Antikörper oder Fragment davon, der/das durch ein Hybridom mit der Zugangsnummer FERM BP-11132 hergestellt wird.

2. Hybridom mit der Zugangsnummer FERM BP-11132.

3. Immunanalytisches Reagens, das bei der Messung von Hunde-CRP und humanem CRP verwendet wird, umfassend das/den monoklonalen Antikörper oder Fragment davon gemäß Anspruch 1.

4. Immunanalytisches Reagens, das bei der Messung von Hunde-CRP und humanem CRP verwendet wird, umfassend ein Latex-Reagens, das mit dem monoklonalen Antikörper oder Fragment davon gemäß Anspruch 1 markiert ist.

5. Immunanalytisches Reagens, das bei der Messung von Hunde-CRP und humanem CRP verwendet wird, umfassend das/den monoklonalen Antikörper oder Fragment davon gemäß Anspruch 1, das/der mit einem Enzym markiert ist.

6. Immunanalytisches Reagens, das bei der Messung von Hunde-CRP und humanem CRP verwendet wird, gemäß Anspruch 5, wobei das Enzym Bacillus subtilis-α-Amylase ist.

7. Trockenes analytisches Element, das bei der Messung von Hunde-CRP und humanem CRP verwendet wird, umfassend das/den monoklonalen Antikörper oder Fragment davon gemäß Anspruch 1, der/das mit einem Enzym markiert ist.

8. Trockenes analytisches Element, das bei der Messung von Hunde-CRP und humanem CRP verwendet wird, gemäß Anspruch 7, wobei das Enzym Bacillus subtilis-α-Amylase ist.

9. Verfahren zur Messung von in einer Probe enthaltenem CRP, welches umfasst, dass einer Probe ermöglicht wird, in Kontakt zu kommen mit dem immunanalytischen Reagens, das bei der Messung von Hunde-CRP und humanem CRP verwendet wird, gemäß mindestens einem der Ansprüche 3 bis 6, oder dem trockenen analytischen Element, das bei der Messung von Hunde-CRP und humanem CRP verwendet wird, gemäß Anspruch 7 und 8.

## Revendications

1. Anticorps monoclonal ou un fragment de celui-ci, qui est produit par un hybridome ayant le No. d'accession FERM BP-11132.

2. Hybridome ayant le No. d'accession FERM BP-11132.

3. Réactif immunoanalytique utilisé dans la mesure de la CRP canine et de la CRP humaine, qui comprend l'anticorps monoclonal ou un fragment de celui-ci selon la revendication 1.

4. Réactif immunoanalytique utilisé dans la mesure de la CRP canine et de la CRP humaine, qui comprend un réactif de latex marqué avec l'anticorps monoclonal ou un fragment de celui-ci selon la revendication 1.

5. Réactif immunoanalytique utilisé dans la mesure de la CRP canine et de la CRP humaine, qui comprend l'anticorps monoclonal ou un fragment de celui-ci selon la revendication 1 qui est marqué avec une enzyme.

6. Réactif immunoanalytique utilisé dans la mesure de la CRP canine et de la CRP humaine selon la revendication 5, où l'enzyme est l'α-amylase de *Bacillus subtilis.*

7. Elément analytique sec utilisé dans la mesure de la CRP canine et de la CRP humaine, qui comprend l'anticorps monoclonal ou un fragment de celui-ci selon la revendication 1 qui est marqué avec une enzyme.

8. Elément analytique sec utilisé dans la mesure de la CRP canine et de la CRP humaine selon la revendication 7, où l'enzyme est l'α-amylase de *Bacillus subtilis.*

9. Procédé de mesure de la CRP contenue dans un échantillon, qui comprend la possibilité pour l'échantillon d'entrer en contact avec le réactif immunoanalytique utilisé dans la mesure de la CRP canine et de la CRP humaine selon l'une quelconque des revendications 3 à 6 ou l'élément analytique sec utilisé dans la mesure de la CRP canine et de la CRP humaine selon les revendications 7 et 8.
